(19) Europäisches Patentamt
European Patent Office
Office européen des brevets

(11) **EP 2 739 313 B1**

(12) **FASCICULE DE BREVET EUROPEEN**

(45) Date de publication et mention
de la délivrance du brevet:
**08.06.2022 Bulletin 2022/23**

(21) Numéro de dépôt: **12750473.6**

(22) Date de dépôt: **03.08.2012**

(51) Classification Internationale des Brevets (IPC):
**A61K 47/10** (2017.01)   **A61K 47/12** (2006.01)
**A61K 47/02** (2006.01)   **A61K 47/32** (2006.01)
**A61K 47/34** (2017.01)

(52) Classification Coopérative des Brevets (CPC):
**A61K 9/0056; C07D 493/04;** A61J 1/10;
B65B 31/02

(86) Numéro de dépôt international:
**PCT/FR2012/051842**

(87) Numéro de publication internationale:
**WO 2013/021126 (14.02.2013 Gazette 2013/07)**

(54) **DIANHYDROHEXITOLS CONDITIONNES DE HAUTE STABILITE CHIMIQUE**

DIANHYDROHEXITOL MIT ERHÖHTER CHEMISCHER STABILITÄT

DIANHYDROHEXITOL WITH INCREASED CHEMICAL STABILITY

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **05.08.2011 FR 1157209**

(43) Date de publication de la demande:
**11.06.2014 Bulletin 2014/24**

(73) Titulaire: **Roquette Frères
62136 Lestrem (FR)**

(72) Inventeurs:
• **IBERT, Mathias
F-59930 La Chapelle D'Armentieres (FR)**
• **WYART, Hervé
F-62149 Cuinchy (FR)**

(74) Mandataire: **Plasseraud IP
66, rue de la Chaussée d'Antin
75440 Paris Cedex 09 (FR)**

(56) Documents cités:
**WO-A2-2009/019371    US-A- 4 529 666**

• **HARA T ET AL: "Isosorbide formulation used as
pharmaceutical and quasi-drug, contains
composition containing crystalline granular
isosorbide having preset moisture content, and
lubricant, packaged with water impermeable
material", WPI/THOMSON, 11 mai 2006
(2006-05-11), XP002473828,**

**Description**

DOMAINE DE L'INVENTION

La présente invention est relative à des dianhydrohexitols conditionnés de telle manière que leur stabilité chimique soit préservée même après un stockage de longue durée. Les dianhydrohexitols conditionnés selon l'invention présentent également la particularité de ne pas être sujets au compactage.

ART ANTERIEUR

Les dianhydrohexitols, également appelés isohexides, sont des produits de déshydratation interne de sucres hydrogénés en $C_6$ (hexitols) tels que le sorbitol, le mannitol et l'iditol.

Parmi ces sucres hydrogénés doublement déshydratés, l'isosorbide est aujourd'hui celui pour lequel on développe et envisage de développer le plus d'applications industrielles, notamment dans le domaine pharmaceutique, dans le domaine des intermédiaires de synthèse chimique et dans le domaine des matières plastiques.

La constatation que les dianhydrohexitols, et en particulier l'isosorbide, sont des produits chimiquement peu stables est relativement récente.

La Demanderesse a en particulier observé que le stockage de dianhydrohexitols, notamment d'isosorbide, même à l'abri de l'humidité de l'atmosphère, pouvait entraîner une dégradation chimique aboutissant, entre autres, à la formation d'acide formique, acide qui présente une odeur caractéristique désagréable, particulièrement gênante dans des applications pharmaceutiques. Cet acide est par ailleurs générateur de problèmes de coloration lors de la synthèse de polymères.

La Demanderesse a ainsi été amenée à mettre au point des procédés de purification et de stabilisation de dianhydrohexitols décrits notamment dans les demandes de brevets EP 1 287 000 et WO 03/043959.

Cependant, la Demanderesse s'est aperçu par la suite que les durées de conservation des dianhydrohexitols, déterminées dans les conditions des tests de stabilité décrits dans les demandes EP 1 287 000 et WO 03/043959, ne reflétaient qu'imparfaitement la stabilité de ces mêmes produits dans les conditions réelles de transport et de stockage. La Demanderesse a relevé en particulier dans certains cas des concentrations d'acide formique relativement plus importantes à proximité du film de conditionnement en polyéthylène. Cette concentration localement élevée pouvait laisser penser que la dégradation des dianhydrohexitols, et de l'isosorbide en particulier, ne se déroulait pas seulement selon une cinétique intrinsèque, dépendante de la température, mais était aussi liée, entre autres, à l'interaction avec le matériau de conditionnement.

La Demanderesse s'est donc fixée pour but de trouver un conditionnement peu coûteux, permettant de conditionner des compositions de dianhydrohexitols et qui, contrairement au polyéthylène classique utilisé dans des conditions standard, permet un stockage de très longue durée sans détérioration de la stabilité chimique même à proximité du matériau de conditionnement.

La demande de brevet JP 2006-117649 divulgue l'utilisation d'un matériau de conditionnement de type film pour emballer de l'isosorbide dans le but de préserver celui-ci contre l'absorption d'eau, de le maintenir sous forme de poudre fluide et d'empêcher la formation d'agrégats. Le film de conditionnement est défini très vaguement comme étant un film multicouches. Ce document ne mentionne pas, en outre, le problème de l'instabilité chimique des dianhydrohexitols au contact du matériau de conditionnement.

La Demanderesse elle-même a élaboré un conditionnement particulier à base de polymère thermoplastique contenant au moins 0,1% d'au moins un agent anti-oxydant pour emballer des dianhydrohexitols. La Demanderesse a alors testé, dans la demande de brevet WO 2009/019371, différents sachets constitués de divers matériaux de conditionnement. Le test a consisté à introduire 50 g d'isosorbide dans les sachets à tester, à fermer immédiatement et hermétiquement lesdits sachets, puis à placer les sachets fermés dans un deuxième sachet en aluminium comportant un revêtement en polyéthylène et également fermé par soudage afin d'assurer l'étanchéité vis-à-vis de l'atmosphère extérieure. Seul l'emballage particulier contenant au moins 0,1% d'au moins un agent anti-oxydant a permis de prévenir la dégradation chimique des compositions de dianhydrohexitols lors de leur stockage à une température de 50°C pour une durée supérieure à 1,5 mois.

Cependant, de telles solutions techniques nécessitent l'élaboration de matériaux de conditionnement particuliers et laissent irrésolu le problème du compactage des dianhydrohexitols lors de leur stockage en sacs de plusieurs kilogrammes voire plusieurs dizaines de kilogrammes.

De plus, ce type de conditionnement n'est pas adapté aux formes liquides d'isosorbide.

La présente invention a donc pour but principal de fournir un conditionnement simple pour les dianhydrohexitols, ne nécessitant pas l'élaboration de matériaux de conditionnement complexes et permettant cependant de préserver la stabilité chimique des dianhydrohexitols sous quelque forme que ce soit et ceci même pour des stockage de longues durées et/ou sous de fortes chaleurs, i.e. un stockage d'une durée supérieure à 2 mois à une température d'au moins 50°C.

Un autre but particulier de la présente invention consiste à fournir des dianhydrohexitols conditionnés qui ne présentent pas de compactage, même après un stockage de plusieurs semaines et/ou un stockage sous de fortes chaleurs.

Un autre but particulier de la présente invention est également de fournir des compositions de dianhydrohexitols pouvant s'écouler facilement dès la sortie de leur conditionnement, et ne laissant pas ou presque de résidus dans le sachet de conditionnement vidé après usage.

Le document US 4 529 666 A concerne un alcool polyvinylique plastifié contenant un ou plusieurs 1,4-monoanhydrohexitols et/ou un ou plusieurs 1,4-3,6-dianhydrohexitols ainsi que son utilisation pour la production de films.

RESUME DE L'INVENTION

La présente invention a par conséquent pour objet un dianhydrohexitol conditionné dans un matériau de conditionnement imperméable aux gaz caractérisé en ce que la pression partielle de l'oxygène $O_2$ à l'intérieur du conditionnement est comprise entre 0,1 mbar et 10 mbar, préférentiellement comprise entre 0,5 mbar et 5 mbar, et plus préférentiellement encore comprise entre 0,5 mbar et 2 mbar, ledit matériau de conditionnement comprenant au moins une couche à base d'aluminium.

L'invention a en outre pour objet un procédé de conditionnement de dianhydrohexitol comprenant l'introduction dudit dianhydrohexitol dans un matériau de conditionnement imperméable aux gaz, puis la fermeture hermétique dudit conditionnement, ledit procédé étant caractérisé en ce qu'il est réalisé dans une atmosphère présentant une pression partielle d'oxygène comprise entre 0,1 mbar et 10 mbar, préférentiellement entre 0,5 mbar et 5 mbar, et plus préférentiellement encore comprise entre 0,5 mbar et 2 mbar, ledit matériau de conditionnement comprenant au moins une couche à base d'aluminium.

DESCRIPTION DETAILLEE

L'invention est définie par les revendications.

Les dianhydrohexitols (1,4 - 3,6-dianhydro-hexitols) comprennent notamment l'isosorbide (1,4 - 3,6-dianhydro-sorbitol), l'isomannide (1,4 - 3,6 dianhydro-mannitol), l'isoidide (1,4 - 3,6-dianhydro-iditol) et les mélanges d'au moins deux de ces produits. De préférence, le dianhydrohexitol conditionné selon la présente invention comprend de l'isosorbide ou est essentiellement constitué d'isosorbide. Il s'agit de préférence d'une composition dont la teneur en isosorbide est au moins égale à 95 % en poids, plus préférentiellement au moins égale à 97 % en poids et, plus préférentiellement encore, au moins égale à 98,5 % en poids (sec/sec).

Les dianhydrohexitols selon l'invention peuvent notamment être issus de tout procédé de préparation connu de l'homme du métier, par exemple d'une distillation d'un brut réactionnel, d'un produit purifié par cristallisation en phase fondue ou en solvant aqueux ou organique éventuellement suivie d'une fusion, d'une concentration à sec d'une solution de dianhydrohexitols purifiée selon le brevet EP 1 287 000, etc.

La présente invention s'applique aux compositions de dianhydrohexitols tant solides que liquides.

En tant que formes solides, il peut s'agir, par exemple, de distillats refroidis et solidifiés ou de cristaux, l'ensemble de ces produits pouvant notamment se présenter sous forme d'une poudre, de cristaux, d'écailles ou de pastilles. De préférence, le dianhydrohexitol conditionné selon l'invention est sous forme de poudre, de cristaux, d'écailles ou de pastilles.

En tant que formes liquides, il peut s'agir, par exemple, de fondus d'isosorbide, c'est-à-dire d'isosorbide de haute pureté, 98 % à 100 % en poids, maintenu sous forme fondue à une température supérieure ou égale à 63 ± 2°C (à pression atmosphérique) ou d'isosorbide en solution, notamment en solution dans l'eau ou dans un solvant organique, à raison notamment de 50 % à 90 %, préférentiellement de 65 % à 85 % en poids d'isosorbide dissout.

Comme il a été expliqué précédemment, les dianhydrohexitols présentent généralement une forte instabilité chimique, notamment au contact de leur matériau de conditionnement, lorsqu'ils sont stockés sur de longues durées et à température élevée.

De nombreuses solutions techniques ont été envisagées mais celles-ci nécessitaient toujours l'élaboration de matériaux de conditionnement particuliers et laissaient par ailleurs irrésolu le problème de compactage des dianhydro-hexitols lors de leur stockage.

Au cours de ses nombreuses recherches, la Demanderesse a imaginé un conditionnement sous vide pour les dianhydrohexitols. Elle s'est intéressée en particulier aux différents domaines de pression utilisés classiquement dans l'industrie, en particulier dans l'industrie agro-alimentaire et chimique.

Dans la présente demande, le terme « vide » est utilisé pour désigner une pression absolue dans une enceinte donnée inférieure à la pression atmosphérique, c'est-à-dire inférieure à environ 1013 mbar.

Dans la présente invention, les pressions, tant absolue que relative, sont évaluées selon toute technique bien connue de l'homme du métier et ceci au niveau de la mer. La pression absolue, ainsi évaluée au niveau de la mer, est d'environ 1013 mbar. Selon l'altitude et les conditions, l'homme du métier saura adapter les niveaux de pression de la présente invention.

Pour rappel, la pression partielle d'un des gaz constituant un mélange gazeux est égale au pourcentage de ce gaz dans le mélange que multiplie la pression absolue du mélange. Ainsi la somme des pressions partielles est égale à la pression absolue.

$$P\ gaz\ =\ PA\ x\ \%\ gaz$$

P gaz : pression partielle du gaz dans le mélange gazeux
PA : pression absolue (par exemple, au niveau de la mer, 1013 mbar)
% gaz : pourcentage du gaz dans le mélange gazeux

La Demanderesse a initialement supposé qu'une réduction de la pression partielle de l'oxygène $O_2$ à l'intérieur du conditionnement de dianhydrohexitols était susceptible d'augmenter la stabilité desdits dianhydrohexitols. Cependant, la Demanderesse a mis en évidence, à titre d'exemple, que pour permettre à la fois un conditionnement envisageable industriellement et un réel maintien de la stabilité chimique, des pressions partielles très faibles, i.e. de l'ordre de 0,01 à 0,001 mbar, ou, au contraire, relativement élevées, i.e. de l'ordre de 50 à 100 mbar ne sont pas du tout acceptables.

Une des solutions techniques envisageables pour réduire la pression partielle de l'oxygène à l'intérieur d'une enceinte hermétiquement close consiste à établir un vide dans ladite enceinte et donc une pression absolue inférieure à environ 1013 mbar.

Dans la présente invention, la Demanderesse s'est intéressée uniquement à des domaines de pression :

- industriellement acceptables, c'est-à-dire ne nécessitant pas une durée de conditionnement longue et/ou du matériel extrêmement fragile et coûteux comme cela est le cas pour obtenir une pression partielle d'oxygène à l'intérieur du conditionnement notamment inférieure à 0,1 mbar ;
- permettant une réelle stabilité chimique du dianhydrohexitol, ceci n'étant pas le cas des pressions partielles d'oxygène élevées, notamment supérieures à 10 mbar.

La Demanderesse s'est aperçue qu'une réduction de la pression partielle d'oxygène de l'ordre de 50 % (pression partielle d'oxygène d'environ 100 mbar à l'intérieur du conditionnement) dans le conditionnement, tout comme une réduction de l'ordre de 75 % (pression partielle d'oxygène d'environ 50 mbar à l'intérieur du conditionnement), n'a aucune influence sur la stabilité des dianhydrohexitols.

De même, le remplissage des conditionnements avec des dianhydrohexitols sous balayage continu d'azote $N_2$ n'a pas permis de préserver la stabilité chimique des dianhydrohexitols sur une longue durée.

C'est finalement tout à fait par hasard, au cours d'un essai additionnel concernant les emballages hermétiques à l'oxygène que la Demanderesse s'est aperçue qu'il existe un seuil de pression partielle d'oxygène à l'intérieur du conditionnement de dianhydrohexitols en-deçà duquel les dianhydrohexitols présentent une stabilité chimique préservée lors d'un stockage de longue durée et/ou sous de fortes chaleurs.

Par ailleurs, et de manière surprenante, la Demanderesse a mis en évidence que des dianhydrohexitols peuvent être stockés dans un matériau de conditionnement quelconque dès lors qu'il est imperméable aux gaz et que l'atmosphère à l'intérieur du conditionnement présente une pression partielle d'oxygène toute particulière, notamment inférieure ou égale à 10 mbar, préférentiellement inférieure ou égale à 5 mbar, et plus préférentiellement encore inférieure ou égale à 2 mbar. De manière inattendue, un tel mode de conditionnement résout également le problème de l'instabilité chimique des dianhydrohexitols au contact du matériau d'emballage.

Dans la présente invention, le terme « imperméable aux gaz » est utilisé pour désigner les emballages dont la perméabilité à l'oxygène est inférieure ou égale à 0,5 $cm^3/m^2/24$ heures, préférentiellement inférieure ou égale à 0,2 $cm^3/m^2/24$ heures (mesures effectuées à 23°C et 0% d'humidité résiduelle selon la norme ASTM 3985).

La présente invention a par conséquent pour objet un dianhydrohexitol conditionné dans un matériau de conditionnement imperméable aux gaz caractérisé en ce que la pression partielle de l'oxygène $O_2$ à l'intérieur du conditionnement est comprise entre 0,1 mbar et 10 mbar, préférentiellement comprise entre 0,5 mbar et 5 mbar, et plus préférentiellement encore comprise entre 0,5 mbar et 2 mbar, ledit matériau de conditionnement comprenant au moins une couche à base d'aluminium. Dans la présente invention, l'expression « compris entre X et Y » n'exclue pas lesdites bornes X et Y.

Une réduction adéquate de la pression partielle d'oxygène de l'atmosphère à l'intérieur du conditionnement de dianhydrohexitols peut notamment être obtenue grâce à un conditionnement sous vide desdits dianhydrohexitols.

La présente invention a donc également pour objet un dianhydrohexitol conditionné caractérisé en ce que :

- l'atmosphère à l'intérieur du conditionnement présente une pression partielle d'oxygène $O_2$ comprise entre 0,1 mbar et 10 mbar, préférentiellement comprise entre 0,5 mbar et 5 mbar, et plus préférentiellement encore comprise entre 0,5 mbar et 2 mbar, et
- la pression absolue à l'intérieur du conditionnement hermétiquement fermé est comprise entre 0,5 mbar et 50 mbar, préférentiellement comprise entre 2 mbar et 25 mbar, et plus préférentiellement encore comprise entre 2 mbar et 10 mbar.

La Demanderesse a également cherché à résoudre le double problème posé par les dianhydrohexitols, à savoir le problème de leur piètre stabilité chimique et également celui de leur forte propension au compactage (ou mottage). Ainsi, la présente invention a également pour objet un dianhydrohexitol conditionné caractérisé en ce que l'atmosphère à l'intérieur du conditionnement présente, outre une pression partielle d'oxygène réduite, une pression absolue à l'intérieur du conditionnement hermétiquement fermé supérieure ou égale à 250 mbar, préférentiellement supérieure ou égale à 400 mbar, et plus préférentiellement encore supérieure ou égale à 500 mbar.

La présente invention a également pour objet un dianhydrohexitol conditionné caractérisé en ce que l'atmosphère à l'intérieur du conditionnement présente une pression partielle d'oxygène réduite, une pression absolue à l'intérieur du conditionnement hermétiquement fermé supérieure ou égale à 250 mbar et une pression partielle d'azote comprise entre 240 mbar et 1012,9 mbar, préférentiellement comprise entre 390 mbar et 1012,9 mbar, et plus préférentiellement encore comprise entre 490 mbar et 1012,9 mbar.

Au sens de la présente invention, on entend par « pression partielle d'oxygène réduite » une pression partielle d'oxygène $O_2$ comprise entre 0,1 mbar et 10 mbar, préférentiellement comprise entre 0,5 mbar et 5 mbar, et plus préférentiellement encore comprise entre 0,5 mbar et 2 mbar.

Le matériau de conditionnement utilisé pour la présente invention comprend au moins une couche à base d'aluminium et peut être choisi en particulier parmi les emballages à base de copolymère éthylène/alcool vinylique (« EVOH »), de poly(chlorure de vinylidène) (« PVDC »), de polyamide (« PA »), de polyacrylonitrile (« PAN »), de poly(acide glycolique) (« PGA ») et/ou de polyéthylène haute densité (« PEHD »). Le matériau de conditionnement peut également être à base d'aluminium et d'un autre métal approprié, déposé par exemple sur la surface extérieure d'une sache, ou une feuille d'aluminium et d'un autre métal approprié. Le matériau de conditionnement utilisé peut également être choisi parmi les containers IBC (*Intermediate Bulk Containers*) et les containers métalliques assortis ou non d'au moins une sache interne et/ou d'au moins un revêtement époxy. Avantageusement, le matériau de conditionnement peut être constitué d'une association d'au moins deux des matériaux ci-avant cités.

L'épaisseur globale du conditionnement ne joue pas un rôle déterminant dans la présente invention. Il peut en effet s'agir d'un matériau mince et souple, par exemple de type film ou feuille, dont l'épaisseur ne dépasse pas quelques dizaines ou quelques centaines de microns, mais également d'un matériau plus rigide sous forme de récipient ayant une forme déterminée.

Le conditionnement peut se présenter avantageusement sous la forme de sachets, saches, sacs, fûts, bidons de toutes formes, dimensions et contenances.

A titre d'exemple, le conditionnement peut être une sache (en anglais « liner » ou « *pouch* ») qui, en vue du transport ou du stockage du dianhydrohexitol, peut être déjà contenue ou être destinée à être contenue dans un récipient souple tel qu'un sac en aluminium ou un « big-bag » (ou « Grand Récipient Vrac Souple » = GRVS ou « *Flexible Intermediate Container* » = FIS) en toile ou tissu, ou dans un récipient rigide tel qu'une caisse en carton.

La Demanderesse a obtenu en particulier d'excellents résultats en termes de stabilité au stockage avec un conditionnement « PE + Alu » correspondant à une sache interne en polyéthylène (PE) de 100 $\mu$m d'épaisseur associée à une sache externe consistant en un complexe aluminium (contenant du polyéthylène de 80 $\mu$m d'épaisseur recouvert d'aluminium de 8,5 $\mu$m d'épaisseur).

Le matériau de conditionnement comprend au moins une couche à base d'aluminium.

Dans la présente invention, le dianhydrohexitol est conditionné hermétiquement, c'est-à-dire que le conditionnement, par exemple le sachet, la sache, le sac, le fût, etc., contenant le dianhydrohexitol est fermé, par exemple par thermosoudage ou au moyen d'un lien approprié, de manière à limiter au maximum, et si possible de manière à supprimer, tout échange de gaz entre l'intérieur de le conditionnement et l'atmosphère extérieure.

Les dianhydrohexitols conditionnés selon l'invention peuvent être obtenus en mettant en œuvre différents types de technologie de mise sous vide ou d'inertage. En particulier, des technologies telles que celles impliquant des cuves à l'intérieur desquelles un vide peut être établi ou celles permettant un inertage direct des conditionnements et, éventuellement, un remplissage sous atmosphère inerte. A titre d'exemple, on peut citer l'azote $N_2$ en tant que gaz d'inertage.

Selon un mode préféré de la présente invention, l'équipement de mise sous vide peut être une cuve tunnel commercialisée par la société BERNHARDT. Un tel équipement est constitué d'une cuve dont la pression absolue interne peut être abaissée à l'aide d'une pompe à vide, et où, éventuellement, la pression absolue interne peut par la suite être remontée à l'aide d'un système de ré-injection de gaz, notamment d'azote, et d'un système automatique, à l'intérieur de la cuve, de fermeture hermétique des conditionnements.

Le conditionnement contenant les dianhydrohexitols peut ainsi être introduit dans la cuve qui est alors fermée hermétiquement. La pression absolue à l'intérieur de la cuve est abaissée, passant ainsi de la pression atmosphérique, c'est-à-dire d'environ 1013 mbar, à une pression plus basse, à titre d'exemple, à une pression absolue interne comprise entre 0,1 mbar et 10 mbar. Puis, la pression absolue au sein de l'enceinte close peut éventuellement être remontée par introduction d'un gaz inerte, notamment d'azote d'une pureté supérieure ou égale à 99,995%. Le conditionnement est alors hermétiquement fermé puis extrait de la cuve.

La présente invention a donc également pour objet un procédé de conditionnement de dianhydrohexitol comprenant l'introduction dudit dianhydrohexitol dans un matériau de conditionnement imperméable aux gaz, puis la fermeture hermétique dudit conditionnement, ledit procédé étant caractérisé en ce qu'il est réalisé dans une atmosphère présentant une pression partielle d'oxygène comprise entre 0,1 et 10 mbar, préférentiellement comprise entre 0,5 et 5 mbar, et plus préférentiellement encore comprise entre 0,5 et 2 mbar, ledit matériau de conditionnement comprenant au moins une couche à base d'aluminium.

Préalablement à la fermeture hermétique du conditionnement, la faible pression absolue à l'intérieur de la cuve résultant de la mise sous vide peut être compensée par l'introduction dans ladite cuve d'un gaz inerte. Ainsi, la présente invention a également pour objet un procédé de conditionnement de dianhydrohexitol tel que l'étape de fermeture du conditionnement est réalisée à une pression comprise entre 250 et 1013 mbar, préférentiellement entre 400 mbar et 1013, et plus préférentiellement entre 500 et 1013 mbar.

Les dianhydrohexitols de la présente invention peuvent être issus d'une distillation d'un brut réactionnel, d'un produit purifié par cristallisation en phase fondue ou en solvant aqueux ou organique éventuellement suivie d'une fusion, d'une concentration à sec d'une solution de dianhydrohexitols purifiée selon le brevet EP 1 287 000.

Les dianhydrohexitols conditionnés conformes à l'invention présentent avantageusement une stabilité au stockage à 50°C et à 80°C dans une étuve sèche, évaluée selon le test A, supérieure à 1 mois, préférentiellement supérieure à 2 mois, plus préférentiellement encore supérieure à 3 mois.

Le test de stabilité A consiste à évaluer dans un premier temps le pH d'un échantillon de produit dissous à 40% en poids de matière sèche dans de l'eau osmosée. On introduit ensuite 50 g d'un autre échantillon de ce même produit dans un matériau de conditionnement à tester et l'on conditionne l'échantillon selon le procédé de la présente invention. On place ensuite le produit conditionné dans une étuve ventilée, thermostatée à une température de 50°C ou 80°C. Plusieurs conditionnements, remplis du même produit, sont placés dans l'étuve. Après une période déterminée, la totalité de l'échantillon de produit est extraite des matériaux de conditionnement et est dissoute à 40 % en poids de matière sèche dans de l'eau osmosée. La mesure de pH est effectuée sur un pHmètre de marque RADIOMETER ANALYTICAL PHM 220 équipé d'une électrode combinée à fil Ag/AgCl de marque METTLER TOLEDO, préalablement étalonnée à l'aide de solutions tampon pH 7 et 4.

Les dianhydrohexitols conditionnés conformes à l'invention présentent avantageusement un pH, évalué selon le test A, supérieur ou égal à 6,0, préférentiellement compris entre 6,0 et 8,1, après au moins 1 mois de stockage dans une étuve ventilée, thermostatée à une température de 50 °C ou de 80°C. Ce pH supérieur ou égal à 6,0 montre une absence de génération d'acidité (synonyme de dégradation des dianhydrohexitols avec formation d'acide formique) et met en évidence une excellente stabilité des dianhydrohexitols conditionnés conformément à l'invention.

Les dianhydrohexitols conditionnés conformes à l'invention ne présentent avantageusement pas de compactage même après un stockage de longue durée à température élevée. Les dianhydrohexitols conditionnés conformément à l'invention présentent donc avantageusement, à la sortie de leur conditionnement, un profil d'écoulement tel que déterminé selon le test B équivalent à celui que ces mêmes dianhydrohexitols présentaient immédiatement après leur fabrication.

Le test d'écoulement B est réalisé en utilisant la tamiseuse de laboratoire VS 1000 commercialisée par la société RETSCH, en suivant la méthode préconisée dans le mode d'emploi de ladite tamiseuse. Pour la réalisation du test d'écoulement B, ladite tamiseuse est équipée d'une tour de tamisage constituée de 3 tamis de 20 cm de diamètre dont la maille est respectivement de 20000 $\mu$m, 5000 $\mu$m et 1000 $\mu$m (les tamis sont placés de haut en bas, de la maille la plus large jusqu'à la maille la plus étroite). Brièvement, le test B consiste à introduire une prise d'essai de 200 g de produit au sommet de la tour de tamisage et à démarrer la tamiseuse en mode continu, à une amplitude de vibration de 50%, durant 10 minutes. Après un tamisage de 10 minutes, la tamiseuse est arrêtée et la quantité de produit retenue sur chacun des tamis est quantifiée par pesée. Le résultat du test d'écoulement B est donc exprimé en pourcentage de produit retenu sur chacun des tamis par rapport à la quantité totale de produit introduit en haut de la tour de tamisage. Le produit ne présente pas de compactage après stockage si son profil d'écoulement (comparaison des pourcentages de retenue sur chaque tamis) est similaire à celui du même produit fraîchement fabriqué et donc soumis à une période de stockage.

Les dianhydrohexitols conditionnés selon l'invention ou susceptibles d'être obtenus par le procédé selon l'invention permettent de réaliser des compositions particulièrement adaptées à des domaines aussi diversifiés que la nutraceutique, la pharmacie, la cosmétique, la chimie, les matériaux de construction, les papiers-cartons, les polymères. Ainsi, la présente invention concerne en outre l'utilisation des dianhydrohexitols conditionnés selon l'invention ou obtenues par le procédé selon l'invention pour la fabrication de dérivés de dianhydrohexitols et de polymères contenant au moins un dianhydrohexitol ou un dérivé de celui-ci.

L'invention sera encore mieux comprise à l'aide des exemples qui suivent, lesquels ne se veulent pas limitatifs et font seulement état de certains modes de réalisation et de certaines propriétés avantageuses des dianhydrohexitols conditionnés conformes à l'invention.

EXEMPLES

Exemple 1 : Stabilité de l'isosorbide à l'état solide en fonction de la pression partielle d'oxygène dans le conditionnement

Les échantillons d'isosorbide testés ont été les suivants :

- **P** : isosorbide de pureté 99,5% en poids, sous forme d'écailles ;

- **P pastilles** : isosorbide de pureté 99,5% en poids, sous forme de pastilles (diamètre 4 mm et épaisseur 2 mm).

Les échantillons ont été emballés dans un conditionnement « PE + Alu » correspondant à une sache interne en polyéthylène (PE) de 100 $\mu$m d'épaisseur associée à une sache externe consistant en un complexe aluminium (contenant 80 $\mu$m d'épaisseur de polyéthylène recouverts de 8,5 $\mu$m d'épaisseur d'aluminium).

Le conditionnement a été réalisé dans une cuve tunnel commercialisée par la société BERNHARDT. Le vide initial réalisé dans la cuve tunnel a varié de la pression atmosphérique (P atm : 1013 mbar au niveau de la mer) à une pression de 5 mbar (Pression absolue initiale : colonne 1, tableau 1), ce qui correspond à une pression partielle d'oxygène (P part d'O$_2$, exprimée en mbar) de 212 mbar à 0,5 mbar (colonne 2, tableau 1). La pression absolue finale à l'intérieur de la cuve, avant la thermo-soudure hermétique du conditionnement, a été ramenée à 1013 mbar par ré-injection d'azote d'une pureté d'au moins 99,995%.

Les échantillons conditionnés ont été soumis au test de stabilité A ci-avant décrit (conditions du test A : étuve sèche à 50°C, stockage d'un ou deux mois). Les résultats de pH-métrie obtenus à l'issue du test de stabilité A sont présentés dans le tableau 1.

Tableau 1

| Pression absolue initiale (mbar) | P part d'O2 (mbar) | pH | | | |
|---|---|---|---|---|---|
| | | P | | P pastilles | |
| | | 1 mois | 2 mois | 1 mois | 2 mois |
| P atm | 212 | 3,1 | === | 4,2 | === |
| 400 | 84 | 3,2 | === | 4,1 | === |
| 180 | 38 | 3,3 | === | 3,9 | === |
| 130 | 27 | 3,5 | === | 4,1 | === |
| 90 | 19 | 3,7 | === | 4,2 | === |
| 23 | 5 | 6, 8 | 6,2 | 7,3 | 7,3 |
| 10 | 2 | 6, 8 | 6,7 | 7,3 | 7,2 |
| 5 | 1 | 7,0 | 6, 6 | 7,2 | 7,3 |

Le pH des échantillons conservés dans un environnement dont la pression partielle de l'oxygène est inférieure ou égale à 10 mbar, plus particulièrement inférieure ou égale à 5 mbar, reste globalement stable (pH $\geq$ 6,0) durant au moins deux mois. Une analyse réalisée après deux mois de conditionnement n'a mis en évidence aucune présence de peroxydes, c'est-à-dire de marqueurs de phénomènes d'oxydation et donc d'instabilité.

L'ensemble des échantillons non conditionnés conformément à l'invention présentent après 1 mois de stockage un pH significativement inférieur à 4,5 et une odeur caractéristique d'acide formique.

Cet exemple montre clairement la supériorité, en termes de stabilité chimique, d'un dianhydrohexitol conditionné selon l'invention par rapport aux modes de conditionnement classique où la pression partielle de l'oxygène à l'intérieur du conditionnement est bien supérieure à 10 mbar.

Exemple 2 : Stabilité de l'isosorbide à l'état fondu en fonction de la pression partielle de l'oxygène dans le conditionnement

Les échantillons d'isosorbide testés ont été des échantillons d'isosorbide de pureté 99,5% en poids maintenu à une température d'environ 80°C (**P fondu**). Les échantillons ont été conditionnés dans un conditionnement constitué d'une seule sache d'un complexe aluminium (contenant 80 $\mu$m d'épaisseur de polyéthylène recouverts d'aluminium de 8,5 $\mu$m d'épaisseur).

Le conditionnement a été réalisé dans une cuve tunnel commercialisée par la société BERNHARDT. Le vide initial réalisé dans la cuve tunnel a varié de la pression atmosphérique (P atm) à une pression d'environ 23 mbar (Pression absolue initiale : colonne 1, tableau 2), ce qui correspond à une pression partielle d'oxygène de 212 mbar à 5 mbar (P part en O2 : colonne 2, tableau 2). La pression absolue finale à l'intérieur de la cuve, avant la fermeture hermétique du conditionnement, a été ramenée à 1013 mbar par ré-injection d'azote d'une pureté d'au moins 99,995%.

Les échantillons conditionnés ont été soumis au test de stabilité A ci-avant décrit (conditions du test A : étuve sèche à 80°C, stockage de 15 jours ou d'un mois). Les résultats de pH-métrie obtenus à l'issue du test de stabilité A sont présentés dans le tableau 2.

Tableau 2

| Pression absolue initiale (mbar) | P part d'O2 (mbar) | pH | |
|---|---|---|---|
| | | 15 jours | 1 mois |
| P atm | 212 | 3,1 | === |
| 23 | 5 | 6,3 | 6, 0 |

Le pH des échantillons conservés dans un environnement dont la pression partielle d'oxygène est inférieure à 10 mbar, plus particulièrement de l'ordre de 5 mbar, reste globalement stable (pH $\geq$ 6,0) durant au moins un mois.

En revanche, les échantillons non conditionnés conformément à l'invention présentent après 15 jours de stockage un pH significativement inférieur à 3,5 et une odeur caractéristique d'acide formique.

Cet exemple montre clairement la supériorité, en termes de stabilité chimique, d'un dianhydrohexitol fondu conditionné selon l'invention par rapport aux modes de conditionnement classique où la pression partielle de l'oxygène à l'intérieur du conditionnement est bien supérieure à 10 mbar.

Exemple 3 : Stabilité de l'isosorbide en fonction de la nature du conditionnement

Les échantillons d'isosorbide testés ont été des échantillons d'isosorbide de pureté 99,5% en poids, contenant 0,2% en poids d'eau et sous forme d'écailles.

Les échantillons sont emballés dans différents conditionnement dont la nature est la suivante :

- PE + Alu = conditionnement constitué d'une sache interne en polyéthylène (PE) de 100 $\mu$m d'épaisseur et d'une sache externe consistant en un complexe aluminium (contenant 80 $\mu$m d'épaisseur de polyéthylène recouverts de 8,5 $\mu$m d'épaisseur d'aluminium)
- Alu seul = conditionnement constitué d'une seule sache d'un complexe aluminium (contenant 80 $\mu$m d'épaisseur de polyéthylène recouverts de 8,5 $\mu$m d'épaisseur d'aluminium)
- Ca + Alu = conditionnement constitué d'une première sache polyéthylène additivé carbone de 150 $\mu$m d'épaisseur et d'une seconde sache, externe, consistant en un complexe aluminium (contenant 80 $\mu$m d'épaisseur de polyéthylène recouverts de 8,5 $\mu$m d'épaisseur d'aluminium)
- TyveK + Alu = conditionnement constitué d'une première sache à base de fibres de polyéthylène à haute densité de 100 $\mu$m d'épaisseur et d'une seconde sache, externe, consistant en un complexe aluminium (contenant 80 $\mu$m d'épaisseur de polyéthylène recouverts de 8,5 $\mu$m d'épaisseur d'aluminium)

Le conditionnement a été réalisé dans une cuve tunnel commercialisée par la société BERNHARDT. Le vide initial réalisé dans la cuve tunnel a varié de la pression atmosphérique (P atm) à une pression de 23 mbar, ce qui correspond à une pression partielle d'oxygène de 212 mbar à 5 mbar. La pression absolue finale à l'intérieur de la cuve, avant la fermeture hermétique du conditionnement, a été ramenée à 1013 mbar par ré-injection d'azote d'une pureté d'au moins 99,995%.

En ce qui concerne l'échantillon « PE + Alu / Flux d'azote », il a été soumis à un conditionnement particulier :

- le conditionnement a été effectué dans un environnement standard (pression absolue égale à la pression atmosphérique, pression partielle en oxygène de l'ordre de 212 mbar),

- le remplissage du conditionnement a été effectué sous un flux continu d'azote de manière à chasser le plus d'air ambiant possible de l'intérieur du conditionnement,

- le flux continu d'azote n'a été stoppé qu'après fermeture hermétique du conditionnement.

Les échantillons conditionnés ont été soumis au test de stabilité A ci-avant décrit (conditions du test A : étuve sèche à 50°C, stockage de 1, 2ou 3 mois). Les résultats de pH-métrie obtenus à l'issue du test de stabilité A sont présentés dans le tableau 3.

Tableau 3

| Conditionnement | P part d'O2 (mbar) | pH | | |
|---|---|---|---|---|
| | | 1 mois | 2 mois | 3 mois |
| Alu seul | 212 | 3,5 | === | === |
| Alu seul | 101 | 3,7 | === | === |
| Alu seul | 50 | 3,4 | === | === |
| Alu seul | 5 | 7,5 | 7,4 | 7,4 |
| PE + Alu | 212 | 3,2 | === | === |
| PE + Alu | 101 | 3,3 | === | === |
| PE + Alu | 50 | 3,6 | === | === |
| PE + Alu | 5 | 7,6 | 7,5 | 7,4 |
| PE + Alu | Flux d'azote | 3,2 | === | === |
| Ca + Alu | 212 | 3,2 | === | === |
| Ca + Alu | 101 | 3,4 | === | === |
| Ca + Alu | 5 | 7,5 | 7,3 | 7,4 |
| TyveK + Alu | 212 | 3,3 | === | === |
| TyveK + Alu | 101 | 3,4 | === | === |
| TyveK + Alu | 5 | 7,6 | 7,5 | 7,5 |

Cet exemple démontre clairement que tout type d'emballage peut être utilisé dès lors qu'il est imperméable aux gaz. Ainsi, dès lors que les échantillons sont conditionnés de manière à ce que l'intérieur du conditionnement présente une pression partielle en oxygène $O_2$ inférieure ou égale à 10 mbar, notamment de l'ordre de 5 mbar, ils restent stables à haute température et sur une période d'au moins 3 mois.

Cet exemple démontre également que le remplissage sous balayage continu d'azote d'un conditionnement avec des dianhydrohexitols ne permet de préserver la stabilité chimique des dianhydrohexitols sur une longue durée (PE + Alu / Flux d'azote).

Exemple 4 : Compactage de l'isosorbide en fonction de la pression au sein du conditionnement

Les échantillons testés ont été des échantillons d'isosorbide P de pureté 99,5% en poids, sous forme d'écailles (pH initial = 7,0).

La nature du conditionnement a été la suivante :

- PE + Alu = conditionnement constitué d'une sache interne en polyéthylène (PE) de 100 $\mu$m d'épaisseur et d'une sache externe consistant en un complexe aluminium (contenant 80 $\mu$m d'épaisseur de polyéthylène recouverts de 8,5 $\mu$m d'épaisseur d'aluminium), ou

- Alu seul = conditionnement constitué d'une seule sache d'un complexe aluminium (contenant 80 $\mu$m d'épaisseur de polyéthylène recouverts de 8,5 $\mu$m d'épaisseur d'aluminium).

Le conditionnement a été réalisé dans une cuve tunnel commercialisée par la société BERNHARDT. La pression absolue initiale dans la cuve tunnel (P absolue initiale) a été abaissée à une pression de 24 mbar, ce qui correspond à une pression partielle d'oxygène d'environ 5 mbar. La pression absolue finale à l'intérieur de la cuve (P absolue finale), avant la thermo-soudure hermétique du conditionnement, a éventuellement été remontée (jusqu'à 500 ou 1013 mbar) par réinjection d'azote d'une pureté d'au moins 99,995%.

Les échantillons conditionnés ont été stockés dans une étuve sèche à 50°C sur une période de 1 ou 2 mois. Les résultats des tests de stabilité A et d'écoulement B obtenus à l'issue du stockage sont présentés respectivement dans les tableaux 4 et 5 ci-après.

Tableau 4 (Test A)

| Conditionnement | P absolue | P absolue | pH | |
|---|---|---|---|---|
| | initiale (mbar) | finale (mbar) | 1 mois | 2 mois |
| PE + Alu | 24 | 24 | 6, 8 | 6,9 |
| Alu seul | 24 | 24 | 6,7 | 6, 8 |
| PE + Alu | 24 | 500 | 6,7 | 6, 8 |
| Alu seul | 24 | 500 | 6,7 | 6,7 |
| PE + Alu | 24 | 1013 | 6, 6 | 6,7 |
| Alu seul | 24 | 1013 | 6,9 | 6, 8 |

Tableau 5 (Test B)

| Conditionnement | P absolue initiale (mbar) | P absolue finale (mbar) | % de retenue après 2 mois de stockage | | | |
|---|---|---|---|---|---|---|
| | | | Tamis de maille ($\mu$m) : | | | |
| | | | 20000 | 5000 | 1000 | Non retenu |
| Isosorbide P à t=0 | === | === | 0 | 20,3 | 77,2 | 2,5 |
| PE + Alu | 24 | 24 | 95,6 | 4,4 | 0 | 0 |
| Alu seul | 24 | 24 | 93,7 | 6,3 | 0 | 0 |
| PE + Alu | 24 | 500 | 0 | 25,3 | 73,1 | 1,6 |
| Alu seul | 24 | 500 | 0 | 24,2 | 74,5 | 1,3 |
| PE + Alu | 24 | 1013 | 0 | 20,5 | 77,0 | 2,5 |
| Alu seul | 24 | 1013 | 0 | 20,3 | 77,3 | 2,4 |

Cet exemple démontre clairement que les échantillons conditionnés selon un mode préféré de l'invention, c'est-à-dire avec une pression partielle d'oxygène réduite et une pression absolue à l'intérieur du conditionnement supérieure à 250 mbar, préférentiellement supérieure ou égale à 500 mbar, suite à une réinjection d'azote, restent stables à haute température et sur une période d'au moins 2 mois et ne se compactent pas. En revanche, les échantillons conditionnés sans réinjection d'azote présentent un compactage important après 2 mois de stockage, tout en restant stables.

**Revendications**

1. Dianhydrohexitol conditionné dans un matériau de conditionnement imperméable aux gaz **caractérisé en ce que** la pression partielle de l'oxygène $O_2$ à l'intérieur du conditionnement est comprise entre 0,1 et 10 mbar, préférentiellement comprise entre 0,5 et 5 mbar, et plus préférentiellement encore comprise entre 0,5 et 2 mbar, ledit matériau de conditionnement comprenant au moins une couche à base d'aluminium.

2. Dianhydrohexitol conditionné selon la revendication 1, **caractérisé en ce que** la pression absolue à l'intérieur du conditionnement est supérieure ou égale à 250 mbar, préférentiellement supérieure ou égale à 400 mbar, et plus préférentiellement encore supérieure ou égale à 500 mbar.

3. Dianhydrohexitol conditionné selon l'une quelconque des revendications précédentes, **caractérisé en ce que** la pression partielle de l'azote $N_2$ à l'intérieur du conditionnement est comprise entre 240 et 1012,9 mbar, préférentiellement comprise entre 390 et 1012,9 mbar, et plus préférentiellement encore comprise entre 490 et 1012,9 mbar.

4. Dianhydrohexitol conditionné selon l'une quelconque des revendications précédentes, **caractérisé en ce que** le matériau de conditionnement correspond à une sache interne en polyéthylène de 100 μm d'épaisseur associée à une sache externe consistant en un complexe aluminium contenant du polyéthylène de 80 μm d'épaisseur recouvert d'aluminium de 8,5 μm d'épaisseur

5. Dianhydrohexitol conditionné selon l'une quelconque des revendications précédentes, **caractérisé en ce que** le dianhydrohexitiol comprend de l'isosorbide ou est essentiellement constitué d'isosorbide.

6. Procédé de conditionnement de dianhydrohexitol comprenant l'introduction dudit dianhydrohexitol dans un matériau de conditionnement imperméable aux gaz, puis la fermeture hermétique dudit conditionnement, ledit procédé étant **caractérisé en ce qu'**il est réalisé dans une atmosphère présentant une pression partielle d'oxygène comprise entre 0,1 et 10 mbar, préférentiellement entre 0,5 et 5 mbar, et plus préférentiellement encore comprise entre 0,5 et 2 mbar, ledit matériau de conditionnement comprenant au moins une couche à base d'aluminium.

7. Procédé de conditionnement de dianhydrohexitol selon la revendication 6 **caractérisé en ce que** l'étape de fermeture du conditionnement est réalisée à une pression absolue supérieure ou égale à 250 mbar, préférentiellement supérieure ou égale à 400 mbar, et plus préférentiellement encore supérieure ou égale à 500 mbar.

## Patentansprüche

1. Dianhydrohexitol, verpackt in einem gasundurchlässigen Verpackungsmaterial, **dadurch gekennzeichnet, dass** der Partialdruck des Sauerstoffs $O_2$ im Inneren der Verpackung zwischen 0,1 und 10 mbar, bevorzugt zwischen 0,5 und 5 mbar und noch stärker bevorzugt zwischen 0,5 und 2 mbar liegt, wobei das Verpackungsmaterial wenigstens eine Schicht auf Aluminiumbasis umfasst.

2. Dianhydrohexitol, verpackt nach Anspruch 1, **dadurch gekennzeichnet, dass** der absolute Druck innerhalb der Verpackung größer oder gleich 250 mbar, bevorzugt größer oder gleich 400 mbar und noch stärker bevorzugt größer oder gleich 500 mbar ist.

3. Dianhydrohexitol, verpackt nach einem der vorhergehenden Ansprüche, **dadurch gekennzeichnet, dass** der Stickstoffpartialdruck $N_2$ im Inneren der Verpackung zwischen 240 und 1012,9 mbar, bevorzugt zwischen 390 und 1012,9 mbar und noch stärker bevorzugt zwischen 490 und 1012,9 mbar liegt.

4. Dianhydrohexitol, verpackt nach einem der vorhergehenden Ansprüche, **dadurch gekennzeichnet, dass** das Verpackungsmaterial einer 100 μm dicken Polyethylen-Innenhülle in Verbindung mit einer Außenhülle entspricht, die aus einem Aluminiumkomplex besteht, der 80 μm dickes Polyethylen enthält, das mit 8,5 μm dickem Aluminium überzogen ist.

5. Verpacktes Dianhydrohexitol nach einer der vorhergehenden Ansprüche, **dadurch gekennzeichnet, dass** das Dianhydrohexitol Isosorbid enthält oder im Wesentlichen aus Isosorbid besteht.

6. Verfahren zum Verpacken von Dianhydrohexitol, umfassend das Einbringen des Dianhydrohexitols in ein gasundurchlässiges Verpackungsmaterial und das anschließende luftdichte Verschließen der Verpackung. Das Verfahren ist **dadurch gekennzeichnet, dass** es in einer Atmosphäre mit einem Sauerstoffpartialdruck zwischen 0,1 und 10 mbar, bevorzugt zwischen 0,5 und 5 mbar und noch stärker bevorzugt zwischen 0,5 und 2 mbar, durchgeführt wird, wobei das Verpackungsmaterial wenigstens eine Schicht auf Aluminiumbasis umfasst.

7. Verfahren zum Verpacken von Dianhydrohexitol nach Anspruch 6, **dadurch gekennzeichnet, dass** der Schritt des Verschließens der Verpackung bei einem absoluten Druck von 250 mbar oder mehr, bevorzugt 400 mbar oder mehr und noch stärker bevorzugt 500 mbar oder mehr durchgeführt wird.

## Claims

1. A dianhydrohexitol packaged in a gastight packaging material, **characterized in that** the oxygen $O_2$ partial pressure inside the packaging is between 0.1 and 10 mbar, preferably between 0.5 and 5 mbar and more preferably still between 0.5 and 2 mbar, said packaging material comprising at least one aluminum-based layer.

**2.** The packaged dianhydrohexitol as claimed in claim 1, **characterized in that** the absolute pressure inside the packaging is greater than or equal to 250 mbar, preferably greater than or equal to 400 mbar and more preferably still greater than or equal to 500 mbar.

**3.** The packaged dianhydrohexitol as claimed in either one of the preceding claims, **characterized in that** the nitrogen $N_2$ partial pressure inside the packaging is between 240 and 1012.9 mbar, preferably between 390 and 1012.9 mbar and more preferably still between 490 and 1012.9 mbar.

**4.** The packaged dianhydrohexitol as claimed in any one of the preceding claims, **characterized in that** the packaging material corresponds to an inner liner made of polyethylene with a thickness of 100 $\mu$m in combination with an outer liner consisting of an aluminum complex comprising polyethylene with a thickness of 80 $\mu$m covered with aluminum with a thickness of 8.5 $\mu$m.

**5.** The packaged dianhydrohexitol as claimed in any one of the preceding claims, **characterized in that** the dianhydrohexitol comprises isosorbide or is essentially composed of isosorbide.

**6.** A process for packaging dianhydrohexitol comprising the introduction of said dianhydrohexitol into a gastight packaging material and then the hermetic closing of said packaging, said process being **characterized in that** it is carried out in an atmosphere exhibiting an oxygen partial pressure of between 0.1 and 10 mbar, preferably between 0.5 and 5 mbar and more preferably still between 0.5 and 2 mbar, said packaging material comprising at least one aluminum-based layer.

**7.** The process for packaging dianhydrohexitol as claimed in claim 6, **characterized in that** the stage of closing the packaging is carried out at an absolute pressure of greater than or equal to 250 mbar, preferably of greater than or equal to 400 mbar and more preferably still of greater than or equal to 500 mbar.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1287000 A
- WO 03043959 A
- JP 2006117649 A
- WO 2009019371 A
- US 4529666 A